# FASCICULE DE BREVET EUROPEEN

(11) **EP 3 551 233 B1**
(45) Date de publication et mention de la délivrance du brevet: **14.08.2024**
(21) Numéro de dépôt: 17822379.8
(22) Date de dépôt: 07.12.2017
(51) Int. Cl.: A61L 2/10, A61L 2/18, A61L 2/20, B65B 55/08

(54) **PROCÉDÉ DE DÉCONTAMINATION D'UNE SURFACE EXTERNE D'UNE PRÉFORME EN MATIÈRE THERMOPLASTIQUE**
VERFAHREN ZUR DEKONTAMINATION EINER AUSSENFLÄCHE EINES THERMOPLASTISCHEN VORFORM
METHOD FOR DECONTAMINATING AN OUTER SURFACE OF A THERMOPLASTIC PREFORM

(30) Priorité: 12.12.2016 FR 1662321
(43) Date de publication de la demande: 16.10.2019
(73) Titulaire: Sidel Participations, 76930 Octeville-sur-Mer (FR)
(72) Inventeur: QUETEL, François, 76930 Octeville-sur-mer (FR); YGER, Benjamin, 76930 Octeville-sur-mer (FR); LETELLIER, Sandy, 76930 Octeville-sur-mer (FR); DEMARE, Jérôme, 76930 Octeville-sur-mer (FR)
(74) Mandataire: Sidel Group
(86) Numéro de dépôt international: PCT/FR2017/053432
(87) Numéro de publication internationale: WO 2018/046875

(56) Documents cités:
- EP-A1- 3 069 846
- EP-A1- 3 069 847
- EP-A2- 2 422 954
- WO-A1-03/084818
- WO-A1-2013/092879
- WO-A1-2017/109421
- JP-A- 2009 161 253

## Description

### DOMAINE TECHNIQUE DE L'INVENTION

L'invention concerne un procédé de décontamination d'une surface externe d'une préforme en matière thermoplastique.

### ÉTAT DE LA TECHNIQUE

On connaît de l'état de la technique des exemples de procédé de décontamination d'une préforme en matière thermoplastique destinée à être transformée en un récipient dans une installation de fabrication.

On distingue dans l'état de la technique, d'une part, des procédés de décontamination destinés à la décontamination de la surface interne de la préforme et d'autre part, des procédés de décontamination destinés à la décontamination de la surface externe de la préforme, notamment du col de la préforme.

Le document EP-2.094.312 décrit une installation pour la fabrication de récipients à partir de préformes en matière thermoplastique décontaminées et plus particulièrement un four de conditionnement thermique de préformes équipant une telle installation.

Selon les enseignements de ce document, le four comporte des moyens de décontamination par émission de rayons ultraviolets qui sont agencés sur le parcours de chauffe après une première zone de chauffage, dite de pénétration, des préformes.

On y réalise une décontamination par irradiation d'au moins la surface externe du col de la préforme au moyen d'un rayonnement ultraviolet (UV), par exemple émis par des lampes.

La décontamination du col obtenue au moyen d'un tel rayonnement ultraviolet améliore le degré de décontamination mais n'est toutefois pas totalement satisfaisante.

La décontamination de la surface externe du col obtenue avec un rayonnement ultraviolet est limitée en raison de son caractère superficiel, le rayonnement ultraviolet ne pénétrant pas en profondeur.

En effet, dans l'hypothèse d'un amas de micro-organismes, le problème est que seuls les micro-organismes se trouvant en surface de l'amas, c'est à dire sur le dessus, vont être effectivement irradiés par le rayonnement ultraviolet.

Les micro-organismes se trouvant en surface d'un tel amas forment un écran qui protège du rayonnement ultraviolet les autres micro-organismes de l'amas situés en dessous, lesquels micro-organismes ne sont alors que peu ou pas irradiés.

De plus, il n'est pas possible de garantir avec un rayonnement ultraviolet que la décontamination d'une surface sera totale, à cause des phénomènes d'ombres qui sont susceptibles de se produire, or cela est tout particulièrement le cas avec la surface externe du col d'une préforme.

Le col de la préforme obtenue par moulage par injection présente en effet sa forme définitive, soit celle du col du récipient.

Le col est par exemple muni d'un filetage pour permettre ultérieurement une fermeture du récipient par un bouchon. Par comparaison au corps de la préforme dont la surface externe est lisse, la surface externe du col est par conséquent plus difficile à irradier à cause des phénomènes d'ombres.

L'irradiation avec un rayonnement ultraviolet présente une moindre efficacité sur certains types de micro-organismes comme les moisissures qu'une décontamination chimique obtenue par exemple avec du peroxyde d'hydrogène (H₂O₂).

Lors du conditionnement thermique de la préforme dans un four, le corps seul est chauffé tandis que le col est lui refroidi pour ne pas subir de déformation susceptible de compromettre la fermeture ultérieure du récipient.

Le fait de ne pas pouvoir chauffer le col de la préforme pour activer du peroxyde d'hydrogène constitue un obstacle pour l'Homme du métier. C'est la raison pour laquelle, la décontamination du col est réalisée par irradiation au moyen d'un rayonnement ultraviolet et non pas suivant une décontamination chimique utilisant en outre du peroxyde d'hydrogène.

Le document WO-03/084818 décrit toutefois un procédé de décontamination du col de préformes en matière thermoplastique destinées à la fabrication de récipients dans lequel du peroxyde d'hydrogène est utilisé.

Le procédé de décontamination consiste à faire passer les cols dans un brouillard de peroxyde d'hydrogène puis, immédiatement après, à soumettre les cols mouillés de peroxyde d'hydrogène à l'action d'un rayonnement ultraviolet et cela pendant une durée minimale prédéterminée.

Un tel procédé de décontamination ne donne toutefois pas entière satisfaction pour différentes raisons détaillées ci-après.

La durée minimale prédéterminée d'application du rayonnement ultraviolet n'est en particulier pas compatible avec les cadences de production d'une installation de fabrication de récipients. Selon l'exemple donné dans ce document, la durée est par exemple de l'ordre d'au moins 8 secondes avec une solution de peroxyde d'hydrogène à 1%.

La quantité résiduelle d'agent stérilisant présente sur le récipient final doit être conforme avec la réglementation applicable de sorte qu'un agent stérilisant comme le peroxyde d'hydrogène doit pouvoir être éliminé après la décontamination.

Selon ce procédé de décontamination, la concentration en peroxyde d'hydrogène du brouillard doit donc nécessairement être très faible afin notamment que les récipients fabriqués à partir des préformes décontaminées ne présentent pas une quantité résiduelle de peroxyde d'hydrogène supérieure au seuil autorisé.

La décontamination de la surface externe du col obtenue n'est par conséquent pas satisfaisante.

Un tel procédé de décontamination contribue à augmenter sensiblement la quantité résiduelle de peroxyde d'hydrogène sans toutefois permettre une amélioration significative du degré de décontamination qui soit, notamment par comparaison à une décontamination de la surface externe du col réalisée uniquement par irradiation au moyen d'un rayonnement ultraviolet selon les enseignements du document EP-2.094.312 précité.

La surface externe du col de la préforme n'est de plus pas recouverte de manière uniforme, le brouillard de peroxyde d'hydrogène se déposant sur ladite surface sous la forme de multiples gouttelettes entre lesquelles subsistent toutefois des zones non recouvertes.

D'une manière générale, on recherche à accroître encore et toujours le degré de décontamination et en particulier pour le conditionnement des produits agro-alimentaires.

On recherche donc de nouvelles solutions pour améliorer le degré de décontamination obtenu ainsi que les types de micro-organismes détruits lors des opérations de décontamination d'une surface externe de préforme, notamment mais non exclusivement, la surface externe du col de la préforme.

Par ailleurs, le document JP2009161253 décrit une méthode de stérilisation de préformes, et le document EP2422954 décrit une injection d'un fluide asceptique au travers d'un alésage prévu traversant dans un dispositif de transport de préformes.

Par ailleurs, le document JP2009161253 décrit une méthode de stérilisation de préformes, et le document EP2422954 décrit une injection d'un fluide asceptique au travers d'un alésage prévu traversant dans un dispositif de transport de préformes. Le but de l'invention est de proposer notamment un nouveau procédé de décontamination permettant de résoudre au moins une partie des inconvénients de l'état de la technique et d'améliorer tout particulièrement la décontamination de la surface externe du col d'une préforme afin de fabriquer des récipients présentant un degré de décontamination plus élevé tout en respectant la règlementation en vigueur sur les quantités résiduelles de peroxyde d'hydrogène.

### BREF RESUME DE L'INVENTION

Dans ce but, l'invention propose un procédé de décontamination d'une surface externe d'une préforme en matière thermoplastique comportant un corps creux muni d'un col, ledit procédé de décontamination comportant successivement au moins :
- une étape de traitement consistant à déposer par condensation du peroxyde d'hydrogène sur la surface externe d'au moins le col de la préforme ;
- une étape d'évaporation d'une partie du condensat de peroxyde d'hydrogène, par circulation forcée d'air sur au moins la surface extérieure du col ; et
- une étape d'irradiation consistant à irradier au moins la surface externe du col de la préforme avec au moins un rayonnement ultraviolet pour activer le peroxyde d'hydrogène.

Par comparaison avec la décontamination des cols obtenue par irradiation selon le document EP-2.094.312, le peroxyde d'hydrogène du condensat pénètre au coeur d'un amas de micro-organismes et n'est donc pas limité à une action superficielle comme un rayonnement ultraviolet.

Par comparaison avec une application de peroxyde d'hydrogène sous la forme d'un brouillard selon les enseignements du document WO-03/084818, le dépôt par condensation lors de l'étape de traitement permet de recouvrir de manière uniforme l'ensemble de la surface externe avec un condensat formé d'un film de buée de peroxyde d'hydrogène.

En effet, le brouillard traversé par les préformes du document WO-03/084818 est composé de gouttelettes liquides dès avant la rencontre des préformes. Les conditions de température et de pression du brouillard sont inférieures à la température de condensation. Les gouttelettes liquides sont déjà condensées et en suspension dans le brouillard. Lorsqu'une préforme traverse le brouillard, les gouttelettes en suspension mouillent directement la préforme et s'agglomèrent en gouttes relativement grosse.

Au contraire, dans l'invention, le dépôt de peroxyde d'hydrogène ayant lieu « par condensation sur la surface de la préforme », ledit peroxyde d'hydrogène avant de rencontrer la préforme est à l'état gazeux, c'est-à-dire dans des conditions de température et de pression supérieure à la température de condensation d'H₂O₂. La préforme est à une température inférieure à la température de condensation d'H₂O₂ de sorte que la condensation n'a lieu qu'à l'instant du contact entre le gaz du H₂O₂ et la préforme. L'effet technique de cette différence de mode de dépôt du H₂O₂ dans l'invention, est que l'H₂O₂ déposé est un film de buée sur la préforme, c'est-à-dire que les particules liquides de la buée sont de très petite taille. Comme les particules liquides de buée ne se forment qu'au contact avec la surface de la préforme, il n'y a pas d'agglomération en gouttes plus grosses. La buée forme un film homogène.

Dans le procédé de décontamination selon l'invention, l'activation du condensat de peroxyde d'hydrogène au moyen du rayonnement ultraviolet selon l'étape d'irradiation n'est pas réalisée immédiatement après le dépôt par condensation du film de buée, c'est à dire consécutivement à l'étape de traitement.

Conformément à l'invention, une étape d'évaporation est mise en oeuvre entre lesdites étapes de traitement et d'irradiation.

L'étape d'évaporation permet d'augmenter progressivement la concentration du peroxyde d'hydrogène dans le condensat recouvrant uniformément la surface externe de la préforme, en particulier celle du col. Le fait que l'étape d'évaporation ne relève pas d'une convection naturelle d'air, ou du convoyage des préformes, mais d'une circulation forcée d'air permet de maitriser le niveau d'évaporation sans dépendre des conditions extérieures.

L'augmentation progressive de la concentration en peroxyde d'hydrogène a pour effet d'affaiblir les micro-organismes présents sur la surface et ce faisant d'en augmenter ainsi la vulnérabilité.

Grâce à l'étape d'évaporation, une concentration élevée en peroxyde d'hydrogène est obtenue sans pour autant que ne le soit la concentration initiale du peroxyde d'hydrogène vaporisé et utilisé pour former le condensat sur la surface externe de la préforme.

Par comparaison avec le procédé de décontamination selon le document WO-03/084818, l'étape d'évaporation est une étape essentielle qui permet d'accroître le degré de décontamination sans augmenter pour autant la concentration du peroxyde d'hydrogène.

L'étape d'évaporation permet également d'éliminer une partie du condensat de peroxyde d'hydrogène, initiant un début d'élimination de l'agent stérilisant simultanément à la décontamination en cours. Cela permet de maitriser la quantité d'agent stérilisant résiduel sur les préformes après l'irradiation UV. Toutefois, le fait qu'une partie seulement du condensat soit évaporé permet également de maitriser la quantité d'agent stérilisant soumis à l'irradiation UV.

Avantageusement, les étapes successives du procédé de décontamination selon l'invention provoquent un « stress oxydatif » qui va progressivement croitre tout au long du procédé de décontamination jusqu'à l'étape d'irradiation.

Lors de l'étape d'irradiation, les micro-organismes ont par conséquent été préalablement fragilisés par l'augmentation de la concentration en peroxyde d'hydrogène qui survient lors de l'étape d'évaporation.

Avantageusement, lors de l'étape d'irradiation, l'irradiation de la surface externe par le rayonnement ultraviolet a pour fonction d'apporter l'énergie nécessaire pour activer le peroxyde d'hydrogène.

L'énergie apportée par le rayonnement ultraviolet provoque une réaction de dismutation du peroxyde d'hydrogène (H₂O₂) dont deux molécules (2 H₂O₂) se décomposent en deux molécules d'eau (2 H₂O) et une de dioxygène (O₂).

Au cours de la réaction, des radicaux libres sont créés et ces radicaux libres vont alors détruire les micro-organismes grâce à quoi on obtient une décontamination de la surface externe de la préforme et tout particulièrement celle du col.

L'énergie apportée par le rayonnement ultraviolet permet d'obtenir un changement d'état du condensat de peroxyde d'hydrogène, de l'état liquide à l'état gazeux, pour activer ledit peroxyde d'hydrogène mais également l'éliminer.

Selon d'autres caractéristiques de l'invention :
- l'étape de traitement est réalisée sur au moins le col de la préforme qui présente une température inférieure à la température de condensation du peroxyde d'hydrogène ;
- l'étape d'évaporation est réalisée par un séchage par air pour évaporer au moins une partie du condensat de peroxyde d'hydrogène ;
- l'air utilisé pour le séchage lors de l'étape d'évaporation présente une température inférieure à 60°C, de préférence comprise entre 20 ° C et 55 ° C, avantageusement de l'ordre de 40 ° C ;
- l'air utilisé pour le séchage lors de l'étape d'évaporation est de l'air de refroidissement mis en circulation dans le four pour refroidir au moins le col de la préforme ;
- l'air utilisé pour le séchage lors de l'étape d'évaporation est constitué par de l'air qui, recyclé après avoir été utilisé pour refroidir au moins une partie de la préforme, présente une température supérieure à celle de l'air de refroidissement ;
- le procédé comporte une autre étape de traitement consistant à déposer par condensation du peroxyde d'hydrogène à l'intérieur de la préforme pour décontaminer la surface interne de la préforme ;
- l'étape de traitement d'au moins la surface externe du col de la préforme est réalisée simultanément, avant ou après ladite autre étape de traitement ;
- l'étape d'irradiation est réalisée après au moins une étape de chauffage des corps dans un four de conditionnement thermique de préformes ;
- l'étape d'irradiation est réalisée sur la surface externe de la préforme.

Selon un autre aspect, l'invention porte sur une installation pour la mise en oeuvre du procédé, l'installation comprenant :
- des moyens de convoyage pour transporter la préforme le long d'un parcours de convoyage selon une direction de convoyage,
- un dispositif de décontamination externe conçu pour déposer par condensation du peroxyde d'hydrogène (H₂O₂) sur une surface externe de la préforme,
- des moyens d'évaporation situés en aval du dispositif de décontamination externe selon la direction de convoyage, lesquels moyens d'évaporation sont conçus pour évaporer une partie du condensat de peroxyde d'hydrogène,
- des moyens d'irradiation UV situés en aval des moyens d'évaporation selon la direction de convoyage, lesquels moyens d'irradiation sont conçus pour irradier la surface externe de la préforme.

Selon un mode particulier de réalisation, l'installation comprend en outre un dispositif de décontamination interne conçu pour déposer par condensation du peroxyde d'hydrogène (H₂O₂) sur une surface interne de la préforme, lequel dispositif de décontamination interne est distinct du dispositif de décontamination externe. L'installation comprend un dispositif de préparation de flux gazeux de peroxyde d'hydrogène (H₂O₂) raccordé à la fois au dispositif de décontamination interne et au dispositif de décontamination externe.

Ce mode particulier de réalisation présente l'avantage de permettre d'ajuster séparément la condensation sur la surface interne et sur la surface externe, tout en ayant un seul générateur de flux gazeux de peroxyde d'hydrogène. En effet, la décontamination interne de la préforme doit être optimisée pour qu'à l'issue des opérations d'activation du peroxyde d'hydrogène, le peroxyde d'hydrogène résiduel ait disparu ou soit en quantité inférieure à une quantité maximale tolérée pour l'application visée pour les préformes, telle que devenir des bouteilles de liquide buvables. Cet objectif souhaitable est plus ou moins contraignant selon le mode d'activation du peroxyde d'hydrogène, par infrarouge seul, ou par UV, ou par une combinaison d'infrarouge et d'UV. En revanche, la décontamination externe de la préforme doit être optimisée pour être efficace sur des surfaces non lisses, telle que la surface d'un col fileté. De plus l'activation du peroxyde d'hydrogène sur cette surface externe est rendue plus complexe à optimiser lorsque l'on ne peut pas utiliser d'infrarouge pour activer le peroxyde d'hydrogène, ce qui est le cas des cols de préforme. Le fait de déposer séparément du peroxyde d'hydrogène sur les surfaces internes et externes permet d'optimiser chacune des opérations de dépose en fonction des contraintes spécifiques à ces deux décontaminations.

Le fait d'évaporer une partie du condensat de peroxyde déposé sur la surface externe permet d'augmenter la concentration de peroxyde agissant sur les éventuelles bactéries de sorte que l'activation par UV à froid est plus rapide et efficace.

Avantageusement, les moyens de convoyage sont équipés d'au moins un mandrin de préhension de la préforme à convoyer, les moyens de convoyage présentant une zone de vétissage dans laquelle ledit mandrin est agencé pour pénétrer dans l'ouverture de la préforme à convoyer et occulter la totalité ou la majeur partie d'une ouverture de ladite préforme. Le dispositif de décontamination interne est agencé en amont de la zone de vétissage de manière à agir sur la préforme avant que le mandrin n'occulte l'ouverture de la préforme et/ou le dispositif de décontamination externe est agencé en aval de la zone de vétissage. Cela permet que la dépose de peroxyde d'hydrogène sur la surface externe n'a plus aucune influence sur la dépose de peroxyde sur la surface interne, cela facilite la maitrise séparée des deux décontaminations.

Selon encore un autre aspect, l'invention porte sur un système comprenant une série de préformes et une installation de décontamination de la série de préformes comprenant
- des moyens de convoyage pour transporter les préformes le long d'un parcours de convoyage selon une direction de convoyage,
- un générateur de peroxyde d'hydrogène à l'état gazeux,
- un dispositif de décontamination externe recevant la série de préformes à une température inférieure à la température de condensation du peroxyde d'hydrogène, raccordé au générateur de peroxyde à l'état gazeux et conçu pour déposer par condensation du peroxyde d'hydrogène (H₂O₂) sur une surface externe de la préforme,
- des moyens d'irradiation UV situés en aval du dispositif de décontamination externe selon la direction de convoyage, lesquels moyens d'irradiation sont conçus pour irradier la surface externe de la préforme.

### BREVE DESCRIPTION DES FIGURES

D'autres caractéristiques et avantages de l'invention apparaitront au cours de la lecture de la description détaillée qui va suivre pour la compréhension de laquelle on se reportera aux dessins annexés dans lesquels :
- la figure 1 est une représentation schématique qui illustre un exemple de réalisation d'une préforme, puis successivement les étapes (a), (b) et (c) du procédé de décontamination de la surface externe d'une telle préforme selon l'invention ;
- la figure 2 est une vue de dessus qui représente schématiquement une partie d'une installation de fabrication de récipients comportant un système de décontamination et un four de conditionnement thermique et qui illustre un premier exemple de mise en oeuvre du procédé de décontamination selon l'invention ;
- la figure 3 est une vue de dessus qui représente schématiquement, comme la figure 2, une partie d'une installation de fabrication de récipients et qui illustre un deuxième exemple de mise en oeuvre du procédé de décontamination selon l'invention ;
- la figure 4 est une vue de dessus qui représente schématiquement comme les figures 2 et 3 précédentes une partie d'une installation de fabrication de récipients et qui illustre un troisième exemple de mise en oeuvre du procédé de décontamination selon l'invention ;
- la figure 5 est une vue de dessus qui représente schématiquement comme les figures 2 à 4 précédentes une partie d'une installation de fabrication de récipients et qui illustre un quatrième exemple de mise en oeuvre du procédé de décontamination selon l'invention.

### DESCRIPTION DETAILLEE DES FIGURES

Dans la suite de la description, on adoptera à titre non limitatif les orientations longitudinale, verticale et transversale en référence au trièdre (L, V, T) représenté sur les figures.

On utilisera également à titre non limitatif les termes « avant » et « arrière » en référence à l'orientation longitudinale, ainsi que « supérieur » et « inférieur » ou « haut » et « bas » en référence à l'orientation verticale et enfin « gauche » et « droite » en référence à l'orientation transversale.

Les termes « intérieur » ou « extérieur » et « interne » ou « externe » sont respectivement utilisés par rapport à la préforme ou au récipient comportant un corps creux et d'une manière générale pour désigner un élément situé soit dedans, soit en dehors de la préforme ou du récipient.

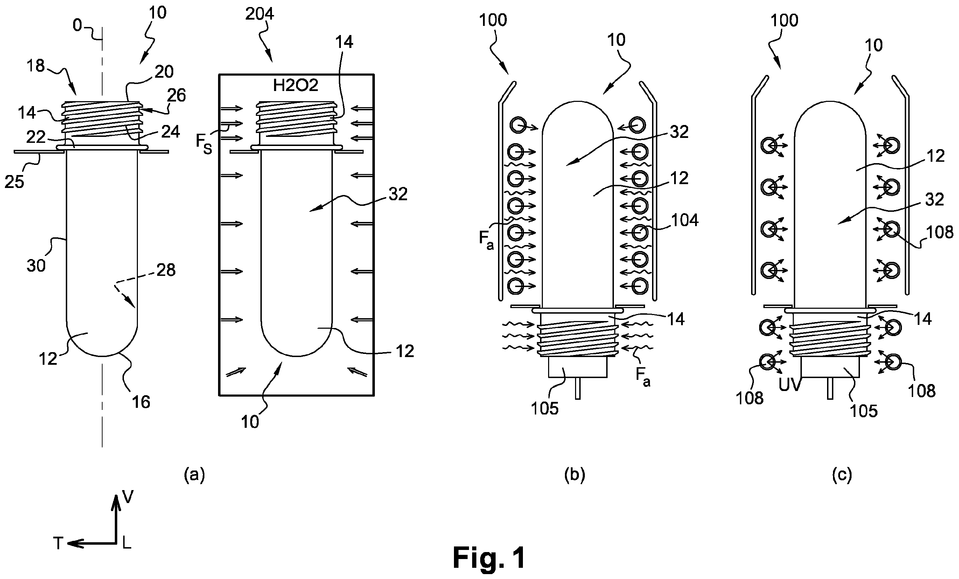

On a représenté sur la figure 1 un exemple de réalisation d'une préforme 10 en matière thermoplastique, ladite préforme 10 illustrant ensuite respectivement les étapes (a) ; (b) et (c) successives du procédé de décontamination d'une surface externe de préforme en matière thermoplastique selon l'invention.

Selon l'exemple représenté sur la figure 1, la préforme 10 comporte principalement un corps 12 creux qui, muni d'un col 14, s'étend suivant un axe O d'orientation verticale.

Le corps 12 de la préforme 10 est fermé à une extrémité par un fond 16 et comporte, à l'extrémité opposée, une ouverture 18 délimitée par un bord 20 (encore appelé buvant) du col 14.

De préférence, la préforme 10 comporte une collerette 22 qui s'étend radialement en saillie vers l'extérieur, au niveau de la jonction du col 14 avec le corps 12.

Une telle préforme 10 est préalablement fabriquée par moulage par injection de matière thermoplastique, en particulier en PET (PolyEthylène-Terephtalate), de sorte que le col 14 présente sa forme définitive, soit celle du col du récipient.

Dans l'exemple, le col 14 est muni d'un filetage 24 afin de permettre une fermeture ultérieure du récipient par un bouchon à vis complémentaire.

Sur la figure 1, la préforme 10 occupe une position dite « col en haut ». La préforme 10 est supportée par l'intermédiaire de moyens 25 de support sur lesquels la face inférieure de la collerette 22 vient prendre appui.

La préforme 10 comporte une gorge 26 annulaire qui est située sur le col 14, entre la collerette 22 et le filetage 24.

La préforme 10 comporte une surface 28 interne et une surface 30 externe qui, respectivement, s'étendent du fond 16 jusqu'au bord 20 du col 14 délimitant l'ouverture 18.

La surface 30 externe de la préforme 10 comporte d'une part une partie correspondant à la surface externe du corps 12 et, d'autre part, une autre partie correspondant à la surface externe du col 14.

Le procédé de décontamination vise plus particulièrement à décontaminer la surface 30 externe de la préforme 10.

La décontamination de la surface 30 externe du col 14 de la préforme 10 est plus particulièrement recherchée en raison, d'une part, de sa proximité avec l'ouverture 18 du col 14 communiquant avec la surface 28 interne et, d'autre part, du fait que le col 14 n'est pas chauffé lors de la fabrication du récipient, par comparaison au corps 12.

Lors du conditionnement thermique de la préforme 10, le chauffage du corps 12 est à une température généralement supérieure à la température de transition vitreuse, par exemple comprise entre 80°C et 100°C. Le chauffage participe à la destruction d'au moins une partie des micro-organismes présents sur la partie de la surface 30 externe correspondant au corps 12, plus particulièrement ceux des micro-organismes qui présentent une vulnérabilité à la chaleur.

Pour les raisons qui viennent d'être indiquées, la décontamination de la surface 30 externe vise donc notamment, mais non exclusivement, à améliorer la décontamination du col 14 de performe 10.

Le procédé de décontamination selon l'invention a pour but de décontaminer la surface 30 externe d'une préforme 10 en matière thermoplastique comportant un corps 12 creux muni d'un col 14.

Dans ce but, le procédé de décontamination comporte successivement au moins :
- une étape (a) de traitement consistant à déposer par condensation du peroxyde d'hydrogène (H₂O₂) sur la surface 30 externe d'au moins le col 14 de la préforme 10 ;
- une étape (b) d'évaporation d'une partie du condensat 32 de peroxyde d'hydrogène (H₂O₂) ; et
- une étape (c) d'irradiation consistant à irradier la surface 30 externe de la préforme 10 avec au moins un rayonnement ultraviolet (UV) pour activer le peroxyde d'hydrogène (H₂O₂).

Par comparaison avec la préforme 10 représentée à gauche sur la figure 1, la préforme 10 adjacente est en cours de traitement lors de l'étape (a) du procédé de décontamination.

Un flux Fs de peroxyde d'hydrogène (H₂O₂) à l'état gazeux est projeté sur la préforme 10 présentant une température inférieure à la température de condensation du peroxyde d'hydrogène (H₂O₂) afin d'obtenir le dépôt par condensation d'un film de buée formant ledit condensat 32.

Avantageusement, la préforme 10 présente une température inférieure au point de rosée du peroxyde d'hydrogène (H₂O₂) de manière à obtenir ledit film de buée.

Le condensat 32 recouvrant la surface externe 30 de la préforme 10 à l'issue de ladite étape (a) étant sous la forme d'un film de buée n'est ainsi pas visible sur la figure 1.

De préférence, la projection du flux Fs est réalisée dans un espace de traitement déterminé, par exemple au moins partiellement confiné par une enceinte comme illustré schématiquement sur la figure 1 à l'étape (a).

L'étape (a) de traitement de la surface 30 externe de la préforme 10 est susceptible d'être réalisée simultanément, avant ou après une autre étape de traitement d'un procédé de décontamination de la surface 28 interne de la préforme 10.

Pour la deuxième étape (b) d'évaporation d'une partie du condensat 32 de peroxyde d'hydrogène (H₂O₂), un séchage par air est par exemple mis en oeuvre pour évaporer une partie du condensat 32 recouvrant la surface 30 externe de la préforme 10.

Un séchage par air est avantageusement utilisé pour l'évaporation du condensat 32 recouvrant la surface 30 externe du col 14 de la préforme.

Un tel séchage par air est également susceptible d'être appliqué pour l'évaporation du condensat 32 recouvrant la surface 30 externe du corps 12 de la préforme 10.

L'évaporation du condensat 32 recouvrant la surface 30 externe du corps 12 de la préforme 10 est également susceptible d'être obtenue par un chauffage par irradiation mis en oeuvre seul ou en combinaison avec un séchage par air.

Avantageusement, l'étape (b) d'évaporation permet d'augmenter progressivement la concentration du peroxyde d'hydrogène dans le condensat 32 recouvrant uniformément la surface 30 externe de la préforme 10.

L'augmentation progressive de la concentration en peroxyde d'hydrogène (H₂O₂) a pour effet d'affaiblir les micro-organismes présents sur la surface 30 externe, d'en augmenter la vulnérabilité, et ce faisant participe à accroître ensuite leur destruction au bénéfice final d'une amélioration de la décontamination.

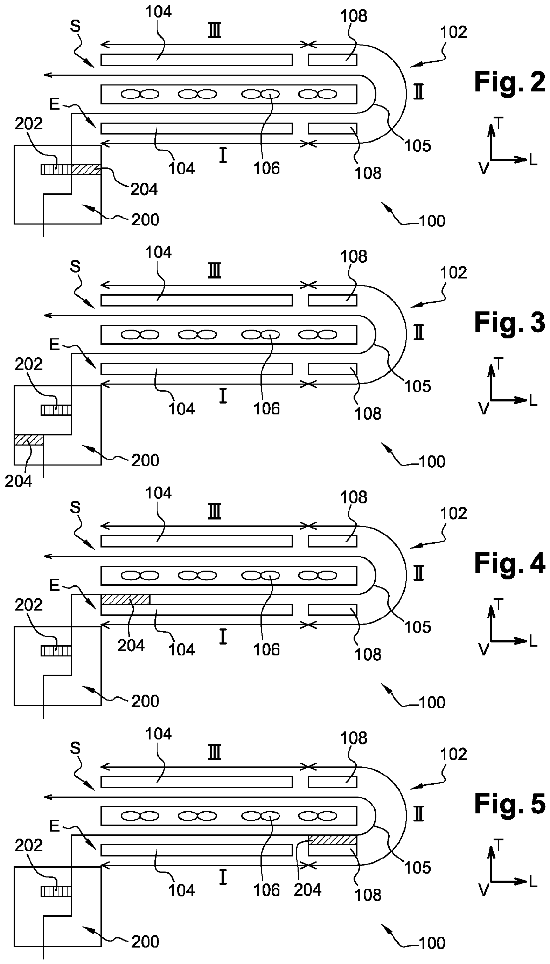

Dans un exemple de réalisation qui sera décrit en détail ultérieurement en référence aux figures 2 à 5, l'air utilisé pour le séchage lors de l'étape (b) d'évaporation est de l'air de refroidissement mis en circulation dans un four de conditionnement thermique de préforme 10.

Avantageusement, l'air utilisé pour le séchage lors de l'étape (b) d'évaporation est constitué par de l'air qui, recyclé après avoir été utilisé pour refroidir au moins une partie de la préforme 10, présente une température supérieure à celle de l'air de refroidissement.

L'air utilisé pour le séchage lors de l'étape (b) d'évaporation du condensat 32 présente une température inférieure à 60°C, de préférence comprise entre 20°C et 55°C.

Avantageusement, la température de l'air de séchage utilisé pour l'étape (b) d'évaporation est de l'ordre de 40°C.

Tel qu'illustré sur la figure 1, l'étape (c) d'irradiation par un rayonnement ultraviolet (UV) est effectuée sur toute la surface 30 externe de la préforme 10 afin d'activer la partie restante, c'est-à-dire non évaporée, du condensat 32 de peroxyde d'hydrogène.

Le procédé de décontamination est susceptible d'être mis en oeuvre dans une installation 100 de fabrication de récipients à partir de préformes 10 en matière thermoplastique.

On a représenté partiellement sur chacune des figures 2 à 5 un exemple d'une telle installation 100 de fabrication de récipients à partir de préformes 10 en matière thermoplastique.

Les figures 2 à 5 sont destinées à illustrer différents exemples de réalisation pour la mise en oeuvre du procédé de décontamination selon l'invention dans une installation 100.

Tel que représenté schématiquement, l'installation 100 comporte au moins un four 102 pour conditionner thermiquement les préformes 10.

Seul les corps 12 des préformes 10 sont conditionnés thermiquement dans le four 102 de l'installation 100 afin d'en ramollir la matière constitutive en vue de leur transformation en récipients.

Les corps 12 des préformes 10 chauffées sont ensuite transformées en récipients dans un machine de moulage (encore appelée souffleuse) que comporte l'installation 100, ladite machine de moulage (non représentée) étant agencée en aval du four 102.

De manière connue, une telle installation 100 comporte des moyens de transfert, tels qu'une ou plusieurs roues comportant par exemple des bras de transfert munis chacun d'une pince de préhension du col, afin d'assurer le transfert d'une préforme 10 ou d'un récipient.

La machine de moulage comporte des unités de moulage réparties circonférentiellement sur un carrousel et destinées chacune à transformer dans un moule, par soufflage ou par étirage-soufflage, au moins une préforme en récipient.

De préférence, l'installation 100 comporte au moins une machine de remplissage (non représentée) des récipients qui est agencée en aval de la machine de moulage. Selon la conception de l'installation 100, une machine d'étiquetage est agencée en aval de la machine de remplissage ou en variante interposée entre la machine de moulage et la machine de remplissage.

Dans l'exemple de réalisation représenté sur les figures 2 à 5, le four 102 est de conception similaire à celui décrit et représenté dans le document EP-2.094.312, document auquel on se reportera avantageusement pour de plus amples détails.

Le four 102 comporte au moins des moyens 104 de chauffage et des moyens 106 de refroidissement associés.

Les moyens 104 de chauffage et les moyens 106 de refroidissement sont disposés sur au moins une partie d'un parcours de chauffe en « U » suivi par les préformes 10, entre une entrée E et une sortie S du four 102.

Le four 102 comporte des moyens 105 de convoyage pour transporter les préformes 10 le long dudit parcours de chauffe.

On connaît des exemples de réalisation de tels moyens 105 de convoyage comportant par exemple au moins une chaîne supportant des moyens de préhension (encore appelés « tournettes ») des préformes 10 par l'intérieur du col 14 et aptes à entraîner individuellement les préformes 10 sur elle-même autour de leur axe O.

De préférence, les préformes 10 sont transportées dans le four 102 dans une position dite « col en bas » par les moyens 105 de convoyage.

A titre d'exemple non limitatif, on pourra se reporter au document WO-00/48819 qui décrit et représente de tels moyens de convoyage de préformes dans un four.

Dans l'exemple, le four 102 comporte ainsi successivement un premier tronçon « I » de chauffage, dit de pénétration, suivi d'un deuxième tronçon « Il », dit de stabilisation, et un troisième tronçon « III » de chauffage, dit de distribution.

Les moyens 104 de chauffage sont par exemple constitués par des lampes à rayonnement infrarouge.

Les moyens 106 de refroidissement sont représentés de manière schématique sous la forme d'une hélice et sont destinés à refroidir les corps 12 et les cols 14 des préformes 10 au moyen d'un flux Fa d'air préalablement filtré.

Le refroidissement par air du corps 12 de chaque préforme 10 est destiné à favoriser un chauffage homogène dans toute l'épaisseur de la paroi de la préforme 10, notamment sans en surchauffer la couche de matière superficielle de la surface 30 externe.

L'air de refroidissement permet d'évacuer la chaleur de convection provoquée par les moyens 104 de chauffage et de favoriser la pénétration des rayonnements infrarouges émis dans l'épaisseur de la matière, en particulier pour établir un gradient à travers le corps 12, entre la surface externe 30 et la surface interne 28.

L'air de refroidissement est également utilisé pour refroidir les cols 14 des préformes 10 afin d'éviter toute déformation qui serait susceptible de compromettre ultérieurement le bouchage du récipient.

On connaît de l'état de la technique différentes conceptions, les moyens 106 de refroidissement du four 102 comportent au moins un circuit de refroidissement par air apte à délivrer un flux Fa d'air de refroidissement aux corps 12 d'une part, et aux cols 14 d'autre part.

Le four 102 comporte encore des moyens 108 d'irradiation aptes à émettre un rayonnement ultraviolet (UV).

Tel qu'illustré sur les figures 2 à 5, les moyens 108 d'irradiation sont disposés sur tout ou partie du deuxième tronçon « Il » de stabilisation du four 102.

Les moyens 108 d'irradiation sont agencés pour irradier au moins la surface 30 externe des cols 14 des préformes 10 et avantageusement également celle des corps 12.

L'installation 100 comporte un système 200 de décontamination d'une préforme 10 en matière thermoplastique.

De préférence, le système 200 de décontamination comporte au moins un dispositif 202 de décontamination, dit « interne », apte à déposer par condensation un film de buée contenant du peroxyde d'hydrogène (H₂O₂) à l'intérieur de chaque préforme 10 en vue d'en réaliser une décontamination de l'ensemble de la surface 28 interne.

Avantageusement, le dispositif 202 de décontamination de la surface 28 interne des préformes 10 est apte à mettre en oeuvre les enseignements du procédé de décontamination selon le document EP-1.896.245.

Le document EP-1.896.245 décrit un procédé de décontamination de la surface interne de préformes, par ailleurs exploité par la Demanderesse sous la marque déposée PREDIS^{®}, et une installation de fabrication de récipients à partir de ces préformes.

Selon une étape initiale, on vérifie que la préforme 10 présente une température qui est inférieure à la température de condensation (Tc) du peroxyde d'hydrogène (H₂O₂) afin d'obtenir une condensation du peroxyde d'hydrogène (H₂O₂).

Les préformes 10 introduites dans le dispositif 202 de décontamination interne du système 200 de décontamination sont à température ambiante et présentent par conséquent une température qui est inférieure à la température de condensation (Tc) du peroxyde d'hydrogène (H₂O₂).

Selon une étape du procédé de décontamination, le dispositif 202 de décontamination projette un jet de vapeur contenant du peroxyde d'hydrogène (H₂O₂) à l'intérieur de la préforme 10 dont la surface 28 interne se recouvre alors d'un condensat formant un film uniforme de buée.

La préforme 10 est ensuite transportée dans le four 102 où le corps 12 de la préforme 10 subit un chauffage par rayonnement émis par les moyens 104 de chauffage du four 102.

Le chauffage appliqué sur le premier tronçon « I » de chauffage permet de porter le corps 12 et l'air contenu à l'intérieur de la préforme 10 à une température supérieure à la température (Ta) d'activation du de peroxyde d'hydrogène (H₂O₂) qui est d'environ soixante-dix degrés Celsius (70°C).

Pour de plus amples détails sur le dispositif 202 de décontamination, on se reportera avantageusement également au document EP-1.896.329 qui décrit une installation pour la fabrication de récipients stériles, notamment des bouteilles, comportant un tel dispositif décontamination de l'intérieur de préformes.

Une décontamination de la surface 30 externe de la préforme 10 est également mise en oeuvre selon l'invention afin de pouvoir garantir une fabrication de récipients avec un degré de décontamination plus élevé.

La décontamination de la surface 30 externe et tout particulièrement celle du col 14 permet d'éviter certains risques de contamination des préformes 10 en maintenant dans l'installation 100 un environnement ultra-propre.

Tel que décrit précédemment en référence à la figure 1, le procédé de décontamination comporte une étape (a) de traitement consistant à déposer par condensation un condensat 32 de peroxyde d'hydrogène (H₂O₂) sur la surface externe 30 de la préforme 10.

Dans le premier exemple de réalisation illustré à la figure 2, le système 200 de décontamination de l'installation 100 comporte un dispositif 204 de décontamination, dit externe, apte à réaliser l'étape (a) de traitement de la surface 30 externe de la préforme 10.

Avantageusement et tel qu'illustré sur la figure 2, l'étape (a) de traitement de la surface 30 externe est réalisée simultanément l'étape de traitement de la surface 28 interne.

Les dispositifs 202 et 204 de décontamination sont regroupés ensemble en amont du four 102 de sorte que les moyens mis en oeuvre pour la préparation du flux (Fs) de peroxyde d'hydrogène (H₂O₂) sont susceptibles d'être mutualisés.

Selon le premier exemple de réalisation de la figure 2, lorsque les préformes 10 sont introduites dans le four 102 par l'entrée E, lesdites préformes 10 comportent d'une part un condensat 32 de peroxyde d'hydrogène (H₂O₂) recouvrant la surface 30 externe et, d'autre part, un autre condensat recouvrant la surface 28 interne.

Les préformes 10 parcourent alors le premier tronçon « I » de chauffage dans lequel est réalisé l'étape (b) d'évaporation d'au moins une partie du condensat 32 de peroxyde d'hydrogène (H₂O₂) recouvrant la surface externe 30 des préformes.

Pour les cols 14 des préformes 10, l'évaporation d'une partie du condensat 32 est obtenue par un séchage par air.

L'air utilisé pour le séchage lors de l'étape (b) d'évaporation présente une température inférieure à 60°C, de préférence comprise entre 20 ° C et 55 ° C, avantageusement de l'ordre de 40°C.

L'air utilisé pour le séchage lors de l'étape (b) d'évaporation est constitué par l'air de refroidissement filtré mis en circulation dans le four 102 pour refroidir notamment les cols 14 des préformes 10.

Pour obtenir un air présentant la température souhaité, l'air utilisé pour le séchage lors de l'étape (b) d'évaporation est par exemple constitué en tout ou en partie par de l'air qui, recyclé après avoir été utilisé pour refroidir au moins une partie de la préforme 10, présente une température supérieure à la température initiale de l'air de refroidissement.

Pour les corps 12 comme pour les cols 14, l'évaporation du condensat 32 sur la surface externe est obtenue par séchage au moyen de l'air de refroidissement tel que cela est illustré sur la figure 1 à l'étape (b) avec le flux Fa d'air.

Pour le corps 12, l'évaporation est également obtenue par le chauffage par rayonnement produit par les moyens 104 de chauffage qui vont participer à l'évaporation d'une partie du condensat 32 recouvrant la surface 30 externe mais aussi provoquer l'évaporation du condensat déposé à l'intérieur par le dispositif 202 de décontamination pour recouvrir la surface 28 interne de la préforme 10.

L'étape (b) d'évaporation est donc réalisée au moins par chauffage pour évaporer au moins une partie du peroxyde d'hydrogène (H₂O₂) déposé sur la surface 30 externe du corps 12 de la préforme 10.

L'étape (c) d'irradiation consistant à irradier la surface externe 30 de la préforme avec au moins un rayonnement ultraviolet (UV) pour activer le peroxyde d'hydrogène (H₂O₂) est réalisée dans le deuxième tronçon « Il » de stabilisation du four 102 dans lequel sont agencés les moyens 108 d'irradiation émettant un rayonnement ultraviolet (UV).

Tel qu'illustré à l'étape (c) sur la figure 1, le rayonnement ultraviolet (UV) émis par les moyens 108 d'irradiation est avantageusement appliqué sur la surface 30 externe de chaque préforme 10.

En variante non représentée, le rayonnement ultraviolet (UV) émis par les moyens 108 d'irradiation est appliqué uniquement sur la surface 30 externe du col 14 de chaque préforme 10.

L'étape (c) d'irradiation permet d'activer le peroxyde d'hydrogène (H₂O₂) dans la partie restante du condensat 32 qui n'a pas été évaporée lors de l'étape (b) d'évaporation.

On décrira ci-après les exemples de mises en oeuvre du procédé de décontamination selon les figures 3 à 5 par comparaison avec la figure 2 qui vient d'être décrite.

Dans l'exemple représenté sur la figure 3, l'étape (a) de traitement de la surface 30 externe par le dispositif 204 de décontamination n'est pas réalisée simultanément mais avant l'étape de traitement de la surface 28 interne de la préforme 10.

Avantageusement, la mutualisation des moyens de préparation du flux Fs de de peroxyde d'hydrogène (H₂O₂) reste possible mais on peut en optimiser l'application en procédant successivement au traitement des cols 14 puis des corps 12 des préformes 10.

Dans l'exemple de la figure 4, l'étape (a) de traitement de la surface 30 externe de la préforme 10 est réalisée après à l'étape de traitement de la surface 28 interne.

Selon cet exemple, l'étape (a) de traitement est réalisée en entrée E du four 102 de conditionnement thermique de préformes 10 de l'installation 100 de fabrication de récipients.

Le dispositif 204 de décontamination est agencé en entrée du parcours de chauffe des préformes 10 dans le four 102 de sorte que l'étape (a) de traitement de la surface 30 externe est réalisée après l'étape de traitement de la surface 28 interne.

Avantageusement, des moyens d'aspiration (non représentés) sont agencés au-dessus de l'espace de traitement dans lequel le flux Fs est projeté pour recouvrir la surface 30 externe du condensat 32 de peroxyde d'hydrogène (H₂O₂).

Dans l'exemple de la figure 5, l'étape (a) de traitement est réalisée après l'étape de traitement de la surface 28 interne et après au moins une étape de chauffage des préformes 10.

Le dispositif 204 de décontamination pour la mise en oeuvre de l'étape (a) de traitement est agencé dans le deuxième tronçon « II » du four 102 de conditionnement thermique de préformes 10.

Dans cet exemple, pour l'étape (a) de traitement, le flux Fs de peroxyde d'hydrogène (H₂O₂) se dépose sous la forme d'un condensat 32 uniquement sur le col 14 de la préforme 10 dès lors que le corps 12 a été chauffé dans le premier tronçon « I » du parcours de chauffe du four 102.

L'étape (a) de traitement est réalisée sur la surface 30 externe du corps 12 de la préforme 10 présentant une température qui est supérieure à la température (Tc) de condensation du peroxyde d'hydrogène (H₂O₂).

Par comparaison dans les exemples des figures 2 à 4, l'étape (a) de traitement provoque un dépôt par condensation du de peroxyde d'hydrogène (H₂O₂) sur la surface 30 externe du corps 12 de la préforme 10 dès lors que le corps 12 présente une température qui est inférieure à la température (Tc) de condensation du peroxyde d'hydrogène (H₂O₂).

## Revendications

1. Procédé de décontamination d'une surface (30) externe d'une préforme (10) en matière thermoplastique comportant un corps (12) creux muni d'un col (14), ledit procédé de décontamination comportant successivement au moins :
- une étape (a) de traitement consistant à déposer par condensation du peroxyde d'hydrogène (H₂O₂) sur la surface (30) externe d'au moins le col (14) de la préforme (10) ;
- une étape (b) d'évaporation d'une partie du condensat (32) de peroxyde d'hydrogène (H₂O₂) par circulation forcée d'air sur au moins la surface extérieure du col ; et
- une étape (c) d'irradiation consistant à irradier au moins la surface (30) externe du col (14) de la préforme (10) avec au moins un rayonnement ultraviolet (UV) pour activer le peroxyde d'hydrogène (H₂O₂).

2. Procédé selon la revendication 1, **caractérisé en ce que** l'étape (a) de traitement est réalisée sur au moins le col (14) de la préforme (10) qui présente une température inférieure à la température (Tc) de condensation du peroxyde d'hydrogène (H₂O₂).

3. Procédé selon la revendication 1 ou 2, **caractérisé en ce que** l'étape (b) d'évaporation est réalisée par un séchage par air pour évaporer au moins une partie du condensat de peroxyde d'hydrogène (H₂O₂).

4. Procédé selon la revendication 3, **caractérisé en ce que** l'air utilisé pour le séchage lors de l'étape (b) d'évaporation présente une température inférieure à 60°C, de préférence comprise entre 20 ° C et 55 ° C, avantageusement de l'ordre de 40°C.

5. Procédé selon la revendication 3 ou 4, **caractérisé en ce que** l'air utilisé pour le séchage lors de l'étape (b) d'évaporation est de l'air de refroidissement mis en circulation dans un four (102) pour refroidir au moins le col (14) de la préforme (10).

6. Procédé selon la revendication 5, **caractérisé en ce que** l'air utilisé pour le séchage lors de l'étape (b) d'évaporation est constitué par de l'air qui, recyclé après avoir été utilisé pour refroidir au moins une partie (12, 14) de la préforme (10), présente une température supérieure à celle de l'air de refroidissement.

7. Procédé selon l'une quelconque des revendications précédentes, **caractérisé en ce que** le procédé comporte une autre étape de traitement consistant à déposer par condensation du peroxyde d'hydrogène (H₂O₂) à l'intérieur de la préforme pour décontaminer la surface (28) interne de la préforme (10).

8. Procédé selon la revendication 7, **caractérisé en ce que** l'étape (a) de traitement d'au moins la surface (30) externe du col (14) de la préforme est réalisée simultanément, avant ou après ladite autre étape de traitement.

9. Procédé selon l'une quelconque des revendications précédentes, **caractérisé en ce que** l'étape (c) d'irradiation est réalisée après au moins une étape de chauffage des corps (12) dans un four (102) de conditionnement thermique de préformes.

10. Procédé selon la revendication 9, **caractérisé en ce que** l'étape (c) d'irradiation est réalisée sur la surface (30) externe de la préforme (10).

11. Installation (100) pour la mise en oeuvre du procédé selon l'une des revendications précédentes, comprenant :
- des moyens (105) de convoyage pour transporter la préforme (10) le long d'un parcours de convoyage selon une direction de convoyage,
- un dispositif (204) de décontamination externe conçu pour déposer par condensation du peroxyde d'hydrogène (H₂O₂) sur une surface (30) externe de la préforme (10),
- des moyens (106) d'évaporation situés en aval du dispositif (204) de décontamination externe selon la direction de convoyage, lesquels moyens (106) d'évaporation comprennent un générateur de circulation forcée d'air sur au moins la surface extérieure du col et sont conçus pour évaporer une partie du condensat de peroxyde d'hydrogène,
- des moyens (108) d'irradiation UV situés en aval des moyens (106) d'évaporation selon la direction de convoyage, lesquels moyens (108 d'irradiation sont conçus pour irradier la surface (30) externe de la préforme (10).

12. Installation selon la revendication 11, comprenant en outre :
- un dispositif (202) de décontamination interne conçu pour déposer par condensation du peroxyde d'hydrogène (H₂O₂) sur une surface interne de la préforme (10), lequel dispositif (202) de décontamination interne est distinct du dispositif (204) de décontamination externe, et
- un dispositif de préparation de flux gazeux de peroxyde d'hydrogène (H₂O₂) raccordé à la fois au dispositif (202) de décontamination interne et au dispositif (204) de décontamination externe.

13. Installation selon la revendication 12, dans laquelle les moyens (105) de convoyage sont équipés d'au moins un mandrin de préhension de la préforme à convoyer, les moyens (105) de convoyage présentant une zone de vétissage dans laquelle ledit mandrin est agencé pour pénétrer dans l'ouverture de la préforme à convoyer et occulter la totalité ou la majeur partie d'une ouverture de ladite préforme (10),
et dans laquelle le dispositif (202) de décontamination interne est agencé en amont de la zone de vétissage de manière à agir sur la préforme (10) avant que le mandrin n'occulte l'ouverture de la préforme.

## Patentansprüche

1. Verfahren zur Dekontamination einer Außenfläche (30) einer Vorform (10) aus einem thermoplastischen Material, die einen Hohlkörper (12) mit einem Hals (14) umfasst, wobei das Dekontaminationsverfahren nacheinander zumindest Folgendes umfasst:
- einen Behandlungsschritt (a), der aus dem Abscheiden mittels Kondensation von Wasserstoffperoxid (H₂O₂) auf der Außenfläche (30) zumindest des Halses (14) der Vorform (10) besteht;
- einen Schritt (b) des Verdampfens eines Teils des Kondensats (32) von Wasserstoffperoxid (H₂O₂) durch eine Zwangsumwälzung von Luft auf zumindest der Außenfläche des Halses und
- einen Bestrahlungsschritt (c), der aus dem Bestrahlen zumindest der Außenfläche (30) des Halses (14) der Vorform (10) mit mindestens einer Ultraviolett- (UV-)Strahlung zur Aktivierung des Wasserstoffperoxids (H₂O₂) besteht.

2. Verfahren nach Anspruch 1, **dadurch gekennzeichnet, dass** der Behandlungsschritt (a) zumindest am Hals (14) der Vorform (10) durchgeführt wird, der eine Temperatur aufweist, die niedriger als die Kondensationstemperatur (Tc) von Wasserstoffperoxid (H₂O₂) ist.

3. Verfahren nach Anspruch 1 oder 2, **dadurch gekennzeichnet, dass** der Verdampfungsschritt (b) durch Lufttrocknen erfolgt, um zumindest einen Teil des Kondensats von Wasserstoffperoxid (H₂O₂) zu verdampfen.

4. Verfahren nach Anspruch 3, **dadurch gekennzeichnet, dass** die Luft, die für das Trocknen beim Verdampfungsschritt (b) verwendet wird, eine Temperatur von weniger als 60 °C, vorzugsweise zwischen 20 °C und 55 °C, vorteilhafterweise in der Größenordnung von 40 °C, aufweist.

5. Verfahren nach Anspruch 3 oder 4, **dadurch gekennzeichnet, dass** es sich bei der Luft, die für das Trocknen beim Verdampfungsschritt (b) verwendet wird, um Kühlluft handelt, die in einem Ofen (102) umgewälzt wird, um zumindest den Hals (14) der Vorform (10) zu kühlen.

6. Verfahren nach Anspruch 5, **dadurch gekennzeichnet, dass** die Luft, die für das Trocknen beim Verdampfungsschritt (b) verwendet wird, aus Luft besteht, die eine Temperatur aufweist, die höher als diejenige der Kühlluft ist, wobei sie zurückgeführt wird, nachdem sie zum Kühlen zumindest eines Teils (12, 14) der Vorform (10) verwendet worden ist.

7. Verfahren nach einem der vorhergehenden Ansprüche, **dadurch gekennzeichnet, dass** das Verfahren einen weiteren Behandlungsschritt umfasst, der daraus besteht, Wasserstoffperoxid (H₂O₂) durch Kondensation im Inneren der Vorform abzuscheiden, um die Innenfläche (28) der Vorform (10) zu dekontaminieren.

8. Verfahren nach Anspruch 7, **dadurch gekennzeichnet, dass** der Schritt (a) zur Behandlung von mindestens einer Außenfläche (30) des Halses (14) der Vorform gleichzeitig mit, vor oder nach dem weiteren Behandlungsschritt erfolgt.

9. Verfahren nach einem der vorhergehenden Ansprüche, **dadurch gekennzeichnet, dass** der Bestrahlungsschritt (c) nach mindestens einem Schritt zum Erwärmen von Körpern (12) in einem Ofen (102) zur thermischen Konditionierung von Vorformen erfolgt.

10. Verfahren nach Anspruch 9, **dadurch gekennzeichnet, dass** der Bestrahlungsschritt (c) auf der Außenfläche (30) der Vorform (10) erfolgt.

11. Anlage (100) zur Durchführung des Verfahrens nach einem der vorhergehenden Ansprüche, die Folgendes umfasst:
- Fördermittel (105) zum Transport der Vorform (10) entlang einer Förderstrecke in einer Förderrichtung,
- eine Vorrichtung (204) zur äußeren Dekontamination, die dazu ausgelegt ist, mittels Kondensation Wasserstoffperoxid (H₂O₂) auf einer Außenfläche (30) der Vorform (10) abzuscheiden,
- Verdampfungsmittel (106), die in der Förderrichtung nach der Vorrichtung (204) zur äußeren Dekontamination angeordnet sind, wobei die Verdampfungsmittel (106) einen Erzeuger einer Zwangsumwälzung von Luft auf mindestens einer Außenfläche des Halses umfassen und dazu ausgelegt sind, einen Teil des Wasserstoffperoxid-Kondensats zu verdampfen,
- UV-Bestrahlungsmittel (108), die in der Förderrichtung nach den Verdampfungsmitteln (106) angeordnet sind, wobei die Bestrahlungsmittel (108) dazu ausgelegt sind, die Außenfläche (30) der Vorform (10) zu bestrahlen.

12. Anlage nach Anspruch 11, die weiterhin Folgendes umfasst:
- eine Vorrichtung (202) zur inneren Dekontamination, die so ausgelegt ist, mittels Kondensation Wasserstoffperoxid (H₂O₂) auf einer Innenfläche der Vorform (10) abzuscheiden, wobei die Vorrichtung (202) zur inneren Dekontamination von der Vorrichtung (204) zur äußeren Dekontamination getrennt ist, und
- eine Vorrichtung zur Herstellung eines Wasserstoffperoxid- (H₂O₂-)Gasstroms, die sowohl mit der Vorrichtung (202) zur inneren Dekontamination als auch mit der Vorrichtung (204) zur äußeren Dekontamination verbunden ist.

13. Anlage nach Anspruch 12, wobei die Fördermittel (105) mit mindestens einem Dorn zum Greifen der zu fördernden Vorform ausgestattet sind, wobei die Fördermittel (105) eine Aufnahmezone aufweisen, in welcher der Dorn so angeordnet ist, dass er in die Öffnung der zu fördernden Vorform eindringt und die Gesamtheit oder einen größeren Teil einer Öffnung der Vorform (10) abdeckt,
und wobei die Vorrichtung (202) zur inneren Dekontamination vor der Aufnahmezone so angeordnet ist, dass sie auf die Vorform (10) einwirkt, bevor der Dorn die Öffnung der Vorform abdeckt.

## Claims

1. Method for decontaminating an outer surface (30) of a preform (10) made of thermoplastic material comprising a hollow body (12) provided with a neck (14), said decontamination method comprising in succession at least:
- a treatment step (a) consisting in depositing by condensation hydrogen peroxide (H₂O₂) on the outer surface (30) of at least the neck (14) of the preform (10);
- a step (b) of evaporation of a part of the condensate (32) of hydrogen peroxide (H₂O₂) by forced air circulation over at least the outer surface of the neck; and
- an irradiation step (c) consisting in irradiating at least the outer surface (30) of the neck (14) of the preform (10) with at least an ultraviolet (UV) radiation to activate the hydrogen peroxide (H₂O₂).

2. Method according to Claim 1, **characterized in that** the treatment step (a) is performed on at least the neck (14) of the preform (10) which has a temperature lower than the condensation temperature (Tc) of the hydrogen peroxide (H₂O₂).

3. Method according to Claim 1 or 2, **characterized in that** the evaporation step (b) is performed by an air drying to evaporate at least a part of the condensate of hydrogen peroxide (H₂O₂).

4. Method according to Claim 3, **characterized in that** the air used for the drying in the evaporation step (b) has a temperature lower than 60°C, preferably lying between 20°C and 55°C, advantageously of the order of 40°C.

5. Method according to Claim 3 or 4, **characterized in that** the air used for the drying in the evaporation step (b) is cooling air circulated in an oven (102) to cool at least the neck (14) of the preform (10).

6. Method according to Claim 5, **characterized in that** the air used for the drying in the evaporation step (b) consists of air which, recycled after having been used to cool at least a part (12, 14) of the preform (10), has a temperature higher than that of the cooling air.

7. Method according to any one of the preceding claims, **characterized in that** the method comprises another treatment step consisting in depositing by condensation hydrogen peroxide (H₂O₂) inside the preform to decontaminate the inner surface (28) of the preform (10) .

8. Method according to Claim 7, **characterized in that** the step (a) of treatment of at least the outer surface (30) of the neck (14) of the preform is performed simultaneously, before or after said other treatment step.

9. Method according to any one of the preceding claims, **characterized in that** the irradiation step (c) is performed after at least a step of heating of the bodies (12) in a preform thermal conditioning oven (102).

10. Method according to Claim 9, **characterized in that** the irradiation step (c) is performed on the outer surface (30) of the preform (10).

11. Installation (100) for implementing the method according to one of the preceding claims, comprising:
- conveying means (105) for transporting the preform (10) along a conveying path in a conveying direction,
- an external decontamination device (204) designed to deposit by condensation hydrogen peroxide (H₂O₂) on an outer surface (30) of the preform (10),
- evaporation means (106) situated downstream of the external decontamination device (204) in the conveying direction, which evaporation means (106) comprise a generator of forced air circulation over at least the outer surface of the neck and are designed to evaporate a part of the condensate of hydrogen peroxide,
- UV irradiation means (108) situated downstream of the evaporation means (106) in the conveying direction, which irradiation means (108) are designed to irradiate the outer surface (30) of the preform (10).

12. Installation according to Claim 11, further comprising:
- an internal decontamination device (202) designed to deposit by condensation hydrogen peroxide (H₂O₂) on an inner surface of the preform (10), which internal decontamination device (202) is distinct from the external decontamination device (204), and
- a hydrogen peroxide (H₂O₂) gaseous flow preparation device coupled both to the internal decontamination device (202) and to the external decontamination device (204).

13. Installation according to Claim 12, wherein the conveying means (105) are equipped with at least one mandrel for gripping the preform to be conveyed, the conveying means (105) having a cladding zone in which said mandrel is arranged to penetrate into the opening of the preform to be conveyed and block all or most of an opening of said preform (10),
and wherein the internal decontamination device (202) is arranged upstream of the cladding zone so as to act on the preform (10) before the mandrel blocks the opening of the preform.
